# EUROPEAN PATENT SPECIFICATION

(11) **EP 3 810 005 B1**
(45) Date of publication and mention of the grant of the patent: **25.10.2023**
(21) Application number: 19823746.3
(22) Date of filing: 17.06.2019
(51) Int. Cl.: A61B 18/14, A61B 18/00, A61B 18/16, A61B 17/221, A61B 17/22

(54) **THROMBECTOMY DEVICES**
THROMBEKTOMIEVORRICHTUNGEN
DISPOSITIFS DE THROMBECTOMIE

(30) Priority: 20.06.2018 US 201862687285 P; 25.02.2019 US 201962809791 P
(43) Date of publication of application: 28.04.2021
(73) Proprietor: Magneto Thrombectomy Solutions Ltd., 6037604 Or Yehuda (IL)
(72) Inventor: TAFF, Yuval, 6299622 Tel Aviv (IL); STERN, Gal, 6937247 Tel Aviv (IL); ORION, Itzhak, 8470110 Beer Sheva (IL)
(74) Representative: Beck Greener LLP
(86) International application number: PCT/IB2019/055032
(87) International publication number: WO 2019/243992

(56) References cited:
- EP-A1- 2 359 764
- WO-A1-00/62851
- WO-A1-2014/025397
- WO-A1-2018/172891
- US-A1- 2001 001 314
- US-A1- 2011 301 594
- US-A1- 2011 308 527
- US-A1- 2014 277 013
- US-A1- 2014 309 579
- US-A1- 2018 116 717
- US-A1- 2018 161 085
- US-B1- 6 730 104

## Description

### FIELD OF THE INVENTION

The present invention relates generally to medical devices for use in procedures, such as thrombectomy procedures for the removal of thrombi from blood vessels.

### BACKGROUND

US Patent 10,028,782 to Orion describes a flexible catheter device capable of being introduced into a body passage and withdrawing fluids therefrom or introducing fluids thereinto. The device includes electrodes configured to apply electrical signals in the body passage for carrying out thrombus dissolution and/or thrombectomy, wherein one of said electrodes is designed to contact the thrombus material and remove it or dissolve it, and wherein the electrical voltage signals are unipolar pulsatile voltage signals.

US Patent Application Publication 2018/0116717 to Taff et al. describes an apparatus for removal of a thrombus from a body of a subject. The apparatus includes a first electrode, made of a first conductive metal, a second electrode, made of a second conductive metal that is different from the first conductive metal, and a voltage source, configured to apply a positive unipolar voltage between the first electrode and the second electrode while the first electrode is in contact with the thrombus, and while the second electrode is inside the body of the subject.

International Patent Application Publication WO/2018/172891 to Taff et al. describes an apparatus that includes an electrically-insulating tube, which includes a distal end having a circumferential wall that is shaped to define one or more perforations, configured for insertion into a body of a subject, an outer electrode, disposed over the distal end of the electrically-insulating tube, and configured to lie at least partly within a thrombus while the electrically-insulating tube is inside the body, and an inner electrode, configured to lie, within the tube, opposite the perforations, while the outer electrode lies at least partly within the thrombus. The outer electrode is configured to attract the thrombus while the outer electrode lies at least partly within the thrombus and the inner electrode lies opposite the perforations, when a positive voltage is applied between the outer electrode and the inner electrode such that electric current flows through the perforations.

International Patent Application Publication WO/2019/102307 to Taff et al. describes an apparatus including a tube. The tube is configured to advance to a blockage and includes a proximal hub configured to connect to a suction-applying device such that, following the advancement of the tube to the blockage, a suction force generated by the suction-applying device is applied, via the tube, to the blockage. The apparatus further includes a shaft, including first and second electrically-conductive circumferential portions, configured to pass through the tube. The apparatus further includes first and second electrically-conductive elements, configured to connect the first and second electrically-conductive circumferential portions to respective terminals of a power source. The first electrically-conductive circumferential portion is configured to attract the blockage when a voltage is applied by the power source, via the first and second electrically-conductive elements, between the first and second electrically-conductive circumferential portions, such that the blockage is anchored to the shaft while the suction force is applied to the blockage.

US 2018/0161085 A1 discloses a bipolar electrode for high frequency thermotherapy. US2018/0116717 A1 discloses a thrombectomy device having first and second electrodes.

### SUMMARY OF THE INVENTION

The invention is defined in claims 1 and 12. Further embodiments of the invention are defined in the dependent claims. Methods are not claimed.

There is provided, in accordance with some embodiments of the present disclosure, an apparatus for removing a blockage from a body of a subject. The apparatus includes a reference electrode, configured for insertion into the body, an electrically-insulative element covering the reference electrode, the electrically-insulative element being shaped to define a gap that exposes a portion of the reference electrode, an active electrode covering the electrically-insulative element, and an electrically-conductive element passing through the reference electrode and electrically connected to the active electrode, the electrically-conductive element being configured to electrically connect the active electrode to a power source such that application, by the power source, of a voltage between the active electrode and the reference electrode causes the active electrode to attract the blockage.

In some embodiments, the active electrode includes a coil.

In some embodiments, the apparatus further includes an electrically-conductive cap connected to a distal end of the active electrode, and the electrically-conductive element is electrically connected to the active electrode by virtue of being connected to the cap.

In some embodiments, the reference electrode includes a coil.

In some embodiments, the reference electrode includes a tube.

In some embodiments, a width of the gap is between 0.01 and 1 mm.

In some embodiments, the gap extends around a full circumference of the reference electrode.

In some embodiments, the gap extends around part of a circumference of the reference electrode such that an angular span of the gap is between 150 and 210 degrees.

In some embodiments, the electrically-insulative element is further shaped to define one or more other gaps that expose one or more other portions of the reference electrode.

In some embodiments, the gap and the other gaps are disposed at different respective positions along a circumference of the reference electrode.

In some embodiments, the gap and the other gaps are disposed at different respective positions along a longitudinal axis of the reference electrode.

In some embodiments, a distance between successive ones of the gaps is greater than 0.1 mm.

In some embodiments, both the reference electrode and the active electrode are attached to the electrically-insulative element.

In some embodiments, the reference electrode is wrapped around the electrically-conductive element.

In some embodiments, the electrically-insulative element includes a tube.

In some embodiments, the active electrode covers the electrically-insulative element both proximally and distally to the gap.

In some embodiments, a length of the active electrode is between 5 and 100 mm.

In some embodiments, a distance between a proximal end of the active electrode and the gap is between 0.5 and 7.5 mm.

In some embodiments, the distance is between 0.5 and 6 mm.

In some embodiments, a distance between a proximal end of the active electrode and the gap is between 5 and 27 mm.

In some embodiments, a distance between a proximal end of the active electrode and the gap is between 2 and 15 mm.

In some embodiments, the distance is between 2 and 12 mm.

In some embodiments, the distance is between 2 and 9 mm.

In some embodiments, a distance between a proximal end of the active electrode and the gap is between 2.5 and 21 mm.

There is further provided, in accordance with some embodiments of the present disclosure, a kit of apparatuses, each of the apparatuses including a reference electrode, configured for insertion into a body of a subject, an electrically-insulative element covering the reference electrode, the electrically-insulative element being shaped to define a gap that exposes a portion of the reference electrode, an active electrode covering the electrically-insulative element, and an electrically-conductive element passing through the reference electrode and electrically connected to the active electrode. Each of the apparatuses has a different respective distance from a proximal end of the active electrode to the gap.

There is further provided, in accordance with some embodiments of the present disclosure, an example method including inserting an apparatus into a conduit within a body of a subject, the apparatus including a reference electrode covered by an electrically-insulative element shaped to define at least one gap that exposes a portion of the reference electrode, and an active electrode covering the electrically-insulative element. The method further includes causing a blockage in the conduit to adhere to the active electrode, by applying a voltage between (a) an electrically-conductive element that passes through the reference electrode and is electrically connected to the active electrode, and (b) the reference electrode. The method further includes, while the blockage adheres to the active electrode, withdrawing the apparatus from the body.

In some embodiments, the conduit includes a blood vessel, and a distance between a proximal end of the active electrode and the gap is 0.5-1.5 times a diameter of the blood vessel.

In some embodiments, the apparatus belongs to a kit of apparatuses, the distance between the proximal end of the active electrode and the gap varying across the apparatuses, and the method further includes:
estimating the diameter of the blood vessel; and
selecting the apparatus from the kit responsively to estimating the diameter.

In some embodiments, a distance between a proximal end of the active electrode and the gap is 0.25-0.75 times a length of the blockage.

In some embodiments, the apparatus belongs to a kit of apparatuses, the distance between the proximal end of the active electrode and the gap varying across the apparatuses, and the method further includes:
estimating the length of the blockage; and
selecting the apparatus from the kit responsively to estimating the length.

There is further provided, in accordance with some embodiments of the present disclosure, an apparatus for removing a blockage from a body of a subject. The apparatus includes a longitudinal element configured for insertion into the body and including a pair of electrodes disposed at different respective positions along a longitudinal axis of the element, the pair including an active electrode having a smaller diameter, and a reference electrode having a larger diameter that is larger than the smaller diameter. The apparatus further includes an electrically-conductive element passing through the longitudinal element and distally connected to a more distal one of the electrodes, the electrically-conductive element being configured to connect the more distal one of the electrodes to a power source outside the body such that, following advancement of the active electrode to the blockage, application of a voltage, by the power source, between the electrically-conductive element and a more proximal one of the electrodes causes the active electrode to attract the blockage.

In some embodiments, the longitudinal element further includes a tube, and the electrically-conductive element passes through the tube.

In some embodiments, the pair of electrodes are coupled distally to the tube.

In some embodiments, the apparatus further includes an electrically-insulative spacer disposed between the pair of electrodes, and a circumference of the spacer is greater at one end of the spacer than at another end of the spacer.

In some embodiments, a circumference of the reference electrode is greater at a distal end of the reference electrode than at a proximal end of the reference electrode.

In some embodiments, the active electrode is cylindrical.

In some embodiments, the more proximal one of the electrodes covers the tube.

In some embodiments, the more proximal one of the electrodes includes an element selected from the group of elements consisting of: a coil, and a mesh.

In some embodiments, the larger diameter is between 1.5 and 7 times larger than the smaller diameter.

In some embodiments, the reference electrode is the more distal one of the electrodes.

In some embodiments, the reference electrode is the more proximal one of the electrodes.

There is further provided, in accordance with some embodiments of the present disclosure, an apparatus for removing a blockage from a body of a subject. The apparatus includes a tube, configured for insertion into the body. The apparatus further includes a pair of electrodes, including an active electrode, and a reference electrode coupled to the tube, the reference electrode being configured to expand outwardly from the tube upon removal of a constraining force. The apparatus further includes an electrically-conductive element distally connected to a more distal one of the electrodes, the electrically-conductive element being configured to connect the more distal one of the electrodes to a power source outside the body such that, following advancement of the active electrode to the blockage and expansion of the reference electrode, application of a voltage, by the power source, between the electrically-conductive element and a more proximal one of the electrodes causes the active electrode to attract the blockage.

In some embodiments, the reference electrode is configured to expand outwardly from the tube such that, following the expansion of the reference electrode, at least part of the reference electrode is opposite the active electrode.

In some embodiments, the reference electrode includes a stent.

In some embodiments, the reference electrode includes a plurality of rods, respective proximal ends of which are coupled to the tube.

In some embodiments, the rods are arranged in a circular arrangement along a circumference of the tube.

In some embodiments, the reference electrode includes between 3 and 12 rods.

In some embodiments, the rods are configured to expand outwardly from the tube such that, following the expansion of the rods, an angle between each of the rods and the tube is between 10 and 60 degrees.

In some embodiments, the reference electrode is coupled to the tube distally to the active electrode, such that the reference electrode is the more distal one of the electrodes.

In some embodiments, the active electrode is coupled to the tube distally to the reference electrode, such that the active electrode is the more distal one of the electrodes.

In some embodiments, the apparatus further includes an electrically-insulative spacer coupled distally to the tube between the active electrode and the tube, such that the active electrode is coupled distally to the tube via the spacer.

In some embodiments, the active electrode is cylindrical.

In some embodiments, the active electrode covers the tube.

In some embodiments, the active electrode includes an element selected from the group of elements consisting of: a coil, and a mesh.

There is further provided, in accordance with some embodiments of the present disclosure, an example method including inserting a longitudinal element, which includes an active electrode and a reference electrode, into a conduit within a body of a subject. The method further includes positioning the active electrode within the conduit such that the active electrode contacts a blockage in the conduit, by contacting an inner wall of the conduit with the reference electrode. The method further includes, subsequently to positioning the active electrode, causing the blockage to adhere to the active electrode, by applying a voltage between the active electrode and the reference electrode, and while the blockage adheres to the active electrode, withdrawing the active electrode from the body.

In some embodiments, positioning the active electrode includes positioning the active electrode within the blockage.

In some embodiments, the conduit includes a blood vessel.

In some embodiments, a diameter of the reference electrode is larger than that of the active electrode.

In some embodiments,
the longitudinal element includes a tube,
the reference electrode is coupled to the tube,
inserting the longitudinal element into the conduit includes inserting the longitudinal element into the conduit while the reference electrode is constrained by a constraining force, and
the method further includes, prior to positioning the active electrode, causing the reference electrode to expand outwardly from the tube by removing the constraining force.

In some embodiments,
the longitudinal element includes a tube,
the reference electrode is coupled to the tube,
inserting the longitudinal element into the conduit includes inserting the longitudinal element into the conduit while the reference electrode is constrained by a constraining force, and
positioning the active electrode includes positioning the active electrode by removing the constraining force such that the reference electrode expands outwardly from the tube and pushes against the inner wall of the conduit.

There is further provided, in accordance with some embodiments of the present disclosure, an apparatus for removing multiple blockages from respective branches of a blood vessel in a body of a subject. The apparatus includes a longitudinal element configured for insertion into the blood vessel, the longitudinal element including a reference electrode. The apparatus further includes multiple electrode-wires configured to extend distally from the longitudinal element into respective ones of the branches, and at least one connecting electrically-conductive element distally connected to the electrode-wires, the connecting electrically-conductive element being configured to connect the electrode-wires to a power source outside the body such that, while the electrode-wires extend into the respective ones of the branches, application of a voltage, by the power source, between the connecting electrically-conductive element and the reference electrode causes the electrode-wires to attract the blockages.

In some embodiments, at least some of the electrode-wires are further configured to bend toward the respective ones of the branches upon removal of a constraining force from the electrode-wires while the electrode-wires are positioned proximally to branches.

In some embodiments, the at least some of the electrode-wires are configured to bend toward the respective ones of the branches by curving toward the respective ones of the branches.

In some embodiments, the longitudinal element includes a tube, and the electrode-wires are coupled distally to the tube.

In some embodiments, the tube includes:
an electrically-conductive portion, including the reference electrode; and
an electrically-insulative portion distal to the electrically-conductive portion.

In some embodiments, the reference electrode covers the tube.

In some embodiments, the connecting electrically-conductive element passes through the tube.

In some embodiments, the electrode-wires are configured to pass through the longitudinal element from outside the body.

In some embodiments, the electrode-wires consist of two electrode-wires.

In some embodiments, the electrode-wires consist of three electrode-wires.

In some embodiments, the electrode-wires include at least four electrode-wires.

There is further provided, in accordance with some embodiments of the present disclosure, an example method for removing multiple blockages from respective branches of a blood vessel in a body of a subject. The method includes inserting multiple electrode-wires into respective ones of the branches, subsequently to inserting the electrode-wires, causing the blockages to adhere to respective ones of the electrode-wires, by applying a voltage between the electrode-wires and a reference electrode positioned proximally to the electrode-wires, and while the blockages adhere to the respective ones of the electrode-wires, withdrawing the electrode-wires from the body.

In some embodiments, the method further includes, prior to inserting the electrode-wires into the respective ones of the branches:
positioning the electrode-wires proximally to the branches; and
while the electrode-wires are positioned proximally to the branches, removing a constraining force from the electrode-wires such that the electrode-wires bend toward the respective ones of the branches.

There is further provided, in accordance with some embodiments of the present disclosure, an apparatus for treating a proximal blockage and a distal blockage in a conduit within a body of a subject. The apparatus includes a longitudinal element configured for insertion into the conduit and including a pair of electrodes, including an active electrode and a reference electrode. The apparatus further includes an expandable element configured to expand within the proximal blockage proximally to the active electrode, and an electrically-conductive element passing through the longitudinal element and distally connected to a more distal one of the electrodes, the electrically-conductive element being configured to connect the more distal one of the electrodes to a power source outside the body such that, following advancement of the active electrode to the distal blockage, application of a voltage, by the power source, between the electrically-conductive element and a more proximal one of the electrodes causes the active electrode to attract the distal blockage.

In some embodiments, the expandable element includes a stent.

In some embodiments, the active electrode is the more distal one of the electrodes.

In some embodiments, the apparatus further includes a tube,

the expandable element is coupled distally to the tube and is configured to detach from the tube when positioned within the proximal blockage, and
the longitudinal element is configured for insertion into the conduit through the tube.

In some embodiments, the expandable element is coupled to the longitudinal element and is configured to detach from the longitudinal element when positioned within the proximal blockage.

In some embodiments, a distance between a distal end of the expandable element and a proximal end of the active electrode is between five and 100 mm.

In some embodiments, the distance is between 30 and 70 mm.

In some embodiments, the expandable element covers the reference electrode.

In some embodiments, the expandable element is coupled to the longitudinal element proximally to the reference electrode.

In some embodiments, a length of the expandable element is between five and 100 mm.

In some embodiments, the length is between 15 and 80 mm.

There is further provided, in accordance with some embodiments of the present disclosure, an example method including inserting, into a conduit within a body of a subject, a longitudinal element, which includes an active electrode and a reference electrode, and an expandable element. The method further includes, while the longitudinal element is within the body, expanding the expandable element within a proximal blockage in the conduit. The method further includes causing a distal blockage in the conduit to adhere to the active electrode, by applying a voltage between the active electrode and the reference electrode, and while the distal blockage adheres to the active electrode, withdrawing the active electrode from the body.

In some embodiments, the conduit includes a blood vessel.

In some embodiments, inserting the expandable element includes inserting the expandable element while the expandable element is coupled to the longitudinal element, and the method further includes detaching the expandable element from the longitudinal element while the expandable element is within the proximal blockage.

In some embodiments, the method further includes, prior to expanding the expandable element, adjusting a distance between a distal end of the expandable element and a proximal end of the active electrode by passing the longitudinal element through the expandable element.

In some embodiments, adjusting the distance includes adjusting the distance responsively to an image of the proximal blockage and the distal blockage.

There is further provided, in accordance with some embodiments of the present disclosure, an apparatus for removing a blockage from a body of a subject. The apparatus includes a wire, an electrically-insulative element partly covering a distal portion of the wire, a coil coiled over the electrically-insulative element, without contacting the wire, such that at least one exposed portion of the wire is disposed between successive windings of the coil or underneath the coil, and an electrically-conductive element distally connected to the coil and configured to connect the coil to a power source outside the body such that, following advancement of the coil to the blockage, application of a voltage, by the power source, between the electrically-conductive element and the wire causes the coil to attract the blockage.

In some embodiments, the electrically-insulative element includes an electrically-insulative strip wound over the distal portion of the wire such that the distal portion of the wire is exposed between at least some windings of the strip.

In some embodiments, a length of the coil is less than 150 mm.

In some embodiments, the length is between five and 50 mm.

In some embodiments, the electrically-conductive element includes a tube, and the wire passes through the tube.

In some embodiments, the apparatus further includes a tube, the wire passes through the tube, and the electrically-conductive element includes another wire that passes through the tube.

There is further provided, in accordance with some embodiments of the present disclosure, an example method including inserting a wire into a conduit within a body of a subject, an electrically-insulative element partly covering a distal portion of the wire, and a coil being coiled over the electrically-insulative element, without contacting the wire, such that at least one exposed portion of the wire is disposed between successive windings of the coil or underneath the coil. The method further includes causing a blockage in the conduit to adhere to the coil, by applying a voltage between the coil and the wire, and while the blockage adheres to the coil, withdrawing the coil from the body.

In some embodiments, the conduit includes a blood vessel.

There is further provided, in accordance with some embodiments of the present disclosure, an apparatus for removing a blockage from a body of a subject. The apparatus includes a longitudinal core, configured for insertion into the body, an active coil and a reference coil, which are both wound around the longitudinal core, and an electrically-insulative element that interposes between the active coil and the reference coil, the active coil being configured to attract the blockage upon application of a voltage, by a power source, between the active coil and the reference coil.

In some embodiments, the longitudinal core includes an insulated wire configured to electrically connect the active coil to the power source.

In some embodiments, the longitudinal core is hollow.

In some embodiments, the electrically-insulative element includes another coil.

In some embodiments, the active coil is wound over the reference coil.

In some embodiments, the active coil and the reference coil are wound next to one another over the longitudinal core.

In some embodiments, an active-coil diameter of the active coil is greater than a reference-coil diameter of the reference coil.

In some embodiments, the active-coil diameter is 1.5-6 times greater than the reference-coil diameter.

In some embodiments, the active-coil diameter is 2-4 times greater than the reference-coil diameter.

In some embodiments, the active coil and the reference coil completely cover the longitudinal core for a length of at least 5 mm.

There is further provided, in accordance with some embodiments of the present disclosure, an example method including inserting an active coil and a reference coil, which are both wound around a longitudinal core, into a conduit within a body of a subject. The method further includes causing a blockage in the conduit to adhere to the active coil, by applying a voltage between the active coil and the reference coil, and while the blockage adheres to the active coil, withdrawing the active coil from the body.

In some embodiments, the conduit includes a blood vessel.

In some embodiments, the method further includes passing a tool through the longitudinal core.

The present invention will be more fully understood from the following detailed description of embodiments thereof, taken together with the drawings, in which:

### BRIEF DESCRIPTION OF THE DRAWINGS

Figs. 1-2 are schematic illustrations of an apparatus, comprising an enlarged reference electrode, for removing a thrombus from a body of a subject, in accordance with some embodiments;
Fig. 3 is a schematic illustration of an apparatus, comprising an expandable reference electrode, for removing a thrombus from a body of a subject, in accordance with some embodiments;
Figs. 4-6 are schematic illustrations of an apparatus for removing multiple blockages from respective branches of a blood vessel in a body of a subject, in accordance with some embodiments;
Fig. 7 is a schematic illustration of an apparatus for treating a proximal blockage and a distal blockage in a blood vessel within a body of a subject, in accordance with some embodiments;
Fig. 8 is a schematic illustration of an apparatus comprising a coiled active electrode for removing a blockage from a body of a subject, in accordance with some embodiments;
Figs. 9A-B are schematic illustrations of an apparatus comprising a coiled active electrode and coiled reference electrode for removing a blockage from a body of a subject, in accordance with some embodiments of the present invention;
Fig. 10 is a schematic illustration of another apparatus for removing a blockage from a body of a subject, in accordance with some embodiments; and
Fig. 11 is a schematic illustration of various transverse cross-sectional cuts through the apparatus shown in Fig. 10, in accordance with some embodiments.

### DETAILED DESCRIPTION OF EMBODIMENTS

### OVERVIEW

Embodiments of the present invention include various devices, each of which is configured to remove at least one blockage from a conduit within the body of a subject by applying an electric field to the blockage. For example, the devices described herein may be used to remove a thrombus or embolus from a blood vessel, or another type of blockage from a digestive tract.

Each of the devices described herein comprises a longitudinal element, typically comprising a flexible tube or wire over the majority of its length. At the distal end of the longitudinal element, the longitudinal element comprises at least one pair of electrodes, referred to herein as an "active electrode" and a "reference electrode," which are configured to connect to different respective terminals of a power source outside the body. In some embodiments, at least one of the electrodes comprises a radiopaque marker to facilitate navigating the device under fluoroscopy. Alternatively or additionally, a radiopaque marker may be disposed on any other portion of the longitudinal element. Typically, the length of the longitudinal element is between 50 and 350 cm.

Each of the devices may be introduced into the subject's body, and may then be advanced, e.g., under fluoroscopy, through the subject's vasculature, until the blockage is reached. Typically, the device is advanced to the blockage within an outer sheath (or "catheter"), which is also referred to herein in certain contexts as a "constraining sheath." Upon the device reaching the blockage, the device is deployed from the sheath by advancing the device from the sheath and/or withdrawing the sheath from over the device. Subsequently, the active electrode is brought into contact with the blockage, or is at least aligned with the blockage.

Next, a positive voltage - typically, a positive unipolar voltage - is applied between the active electrode and the reference electrode, typically for a duration of between 5 and 120 seconds. (In the context of the present application, including the claims, a signal may be described as "positive unipolar" if the signal is positive for at least 80% of the duration of the signal.) The applied voltage causes the active electrode to electrostatically attract the negatively-charged blockage, such that the blockage becomes ionically bonded (or "anchored") to the active electrode. Subsequently to applying the voltage - or, in some cases, while continuing to apply the voltage - the device and the blockage are removed from the subject.

Some devices described herein comprise an enlarged or expandable reference electrode coupled to a tube. By virtue of its enlarged diameter, the reference electrode may contact the inner wall of the blood vessel, thus helping to position the active electrode near the center of blood vessel so as to improve the contact between the active electrode and the blockage.

For example, each of the electrodes may comprise a respective cylinder disposed distally to the tube, the reference-electrode cylinder having a diameter that is greater than that of the active-electrode cylinder. Alternatively, the reference electrode may comprise an expandable element, such as a set of rods made from a shape memory material. Upon the active electrode reaching the blockage, the expandable element is allowed to expand radially outward from the tube, such that the expandable element contacts the inner wall of the blood vessel.

Other devices described herein are configured to simultaneously treat multiple blockages. One such device is configured to treat multiple blockages situated in different respective branches of a blood vessel. The device comprises an active electrode comprising a plurality of wires, which are disposed at the distal end of a tube. The device is advanced through the blood vessel until at least one of the wires enters each of the branches. Optionally, at least two of the wires may be configured to curve or angle in different respective directions, so as to increase the probability that the wires will enter different respective ones of the branches.

Another such device is configured to treat two blockages disposed at different respective axial positions in a blood vessel. The device comprises a detachable, expandable element, such as a stent, coupled to a tube. Upon the expandable element being disposed within the more proximal blockage, the expandable element is allowed to expand and is also detached from the tube, such that the more proximal blockage is pushed against the inner wall of the blood vessel. In addition, the more distal blockage is captured by the active electrode as described above, and the device is then withdrawn through the expandable element.

Yet other devices described herein comprise a coiled active electrode. Advantageously, these devices may provide improved distribution of the electric current between the electrodes.

For example, the reference electrode may comprise a wire that is partly covered by an insulating element, the active electrode being coiled over the insulating element. Gaps in the insulating element may facilitate the flow of electric current (carried by ions) between the two electrodes. Alternatively, the reference electrode may also comprise a coil, which is wound, in parallel with the active-electrode coil, around a plastic tube or another electrically-insulative core. Optionally, to increase the contact area between the active electrode and the blockage, the diameter of the active-electrode coil may be greater than that of the reference-electrode coil.

As yet another alternative, the reference electrode may comprise a tube or coil that is covered by an electrically-insulative element, and the active electrode may be coiled around the electrically-insulative element. The electrically-insulative element may be shaped to define a gap that exposes part of the reference electrode such that, when a voltage is applied between the electrodes, electric current flows through the gap. Advantageously, the gap may be positioned so as to generally maximize the attractive force applied to the thrombus.

In general, each of the electrically-insulative elements described herein may comprise silicone, polyimide, polyurethane, polyethylene (e.g., polyethylene terephthalate), polytetrafluoroethylene, and/or any other suitable biocompatible electrically-insulative material.

In general, the active electrode may comprise any one or more biocompatible materials having a suitably high conductivity and, optionally, visibility under fluoroscopy. Examples of such materials include gold, platinum, and alloys of platinum and iridium. Similarly, the reference electrode may comprise any one or more biocompatible materials having a suitably high conductivity, such as stainless steel, nitinol, or titanium. Typically, the reference electrode has an electronegativity that is less than that of the active electrode. Other electrically-conductive elements described herein, such as wires and tubes, may similarly comprise any suitable biocompatible conductor, such as stainless steel, nitinol, and/or titanium. Wherever relevant, elements may be coated for improved lubricity.

In general, in each of the embodiments described herein, unless specified otherwise, each electrode may have any suitable form. Examples of suitable forms include a hollow or solid cylinder (including, for example, a ring), a tube, a coil, a mesh, and any combination of two or more of the above. In some cases, an electrode may be disposed over the circumference of a tube or another longitudinal element.

For simplicity, the present description mostly relates to devices for the removal of thrombi from blood vessels, and the devices described herein are referred to as "thrombectomy devices." Nevertheless, as noted above, the embodiments described herein may also be used to remove other types of blockages from blood vessels or other types of conduits.

### DEVICES HAVING AN ENLARGED OR EXPANDABLE REFERENCE ELECTRODE

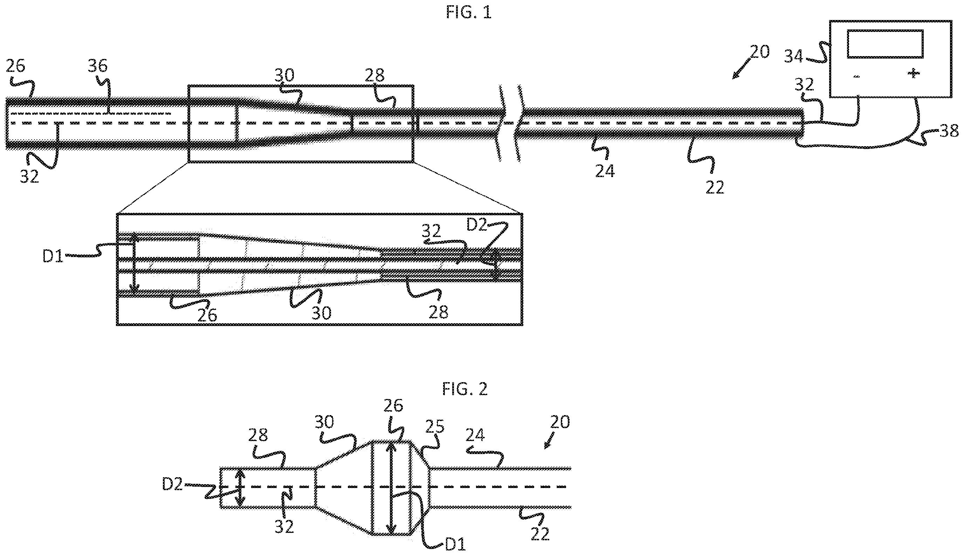

Reference is initially made to Figs. 1-2, which are schematic illustrations of an apparatus 20, comprising an enlarged reference electrode, for removing a thrombus from a body of a subject, in accordance with some embodiments of the present disclosure.

Apparatus 20 comprises a longitudinal element 22 configured for insertion into the body. Longitudinal element 22 comprises a pair of electrodes - in particular, an active electrode 28 and a reference electrode 26 - disposed at different respective positions along a longitudinal axis 36 of the longitudinal element. For example, as shown in Fig. 1, reference electrode 26 may be disposed distally to active electrode 28. Alternatively, as shown in Fig. 2, reference electrode 26 may be disposed proximally to active electrode 28.

Typically (provided that the active electrode is not elongated as described below), the length of the active electrode is less than 150 mm, such as between 5 and 50 mm. Also typically (provided that the reference electrode is not elongated as described below), the length of the reference electrode is less than 50 mm, such as between 5 and 50 mm. In some embodiments, one or both of the electrodes are cylindrical. To facilitate navigating the apparatus, the more distal electrode may be curved, e.g., J-shaped.

Typically, longitudinal element 22 further comprises an electrically-conductive or electrically-insulative tube 24, which is configured to extend to the thrombus from the exterior of the subject's body. In such embodiments, the pair of electrodes may be coupled distally to tube 24, an electrically-insulative spacer 30 being disposed between the pair of electrodes. For example, as shown in Fig. 1, active electrode 28 may be coupled to the distal end of the tube, spacer 30 may be coupled distally to the active electrode, and reference electrode 26 may be coupled distally to the spacer. Typically, the length of the spacer is less than 5 mm (e.g., less than 1 mm), such that a distance of less than 5 mm (e.g., less than 1 mm) separates the reference electrode from the active electrode.

Alternatively, the more proximal electrode and/or the more distal electrode may cover the tube, e.g., such that a distance of less than 5 mm (e.g., less than 1 mm) separates the reference electrode from the active electrode. For example, at least one of the electrodes may comprise a coil (having a constant or variable pitch) that is coiled around tube 24, a mesh (or "braid") that is wrapped around tube 24, or an outer tube that covers tube 24.

In some embodiments, to provide added flexibility to tube 24, the wall of the tube is not entirely solid. For example, the distal portion of the wall of the tube, and/or any other portion of the wall, may be shaped to define a coil or braid. (It is noted that in the context of the present application, including the claims, a "tube" may include any hollow longitudinal structure, regardless of whether the wall of the structure is solid.)

In other embodiments, longitudinal element 22 does not comprise tube 24; rather, the more proximal electrode is elongated, such that the more proximal electrode extends to the thrombus from the exterior of the subject's body. An alternative formulation of the above is that tube 24 itself may function as the more proximal electrode.

Apparatus 20 further comprises a wire 32 (or another electrically-conductive element, such as a trace) passing through the longitudinal element and distally connected to the more distal electrode. For example, as shown in Fig. 1, wire 32 may pass through tube 24, active electrode 28, and spacer 30, with the distal end of the wire connected to the reference electrode. In some embodiments, as shown in Fig. 1, the more distal electrode is hollow; in such embodiments, wire 32 may further pass through the more distal electrode, the distal end of the wire being connected to the distal end of the more distal electrode.

Typically, the entire length of wire 32 passing through the longitudinal element is insulated, with the exception of the distal end of the wire. For example, the wire may comprise an electrically-conductive core, which is covered over most of its length by an electrically-insulative layer of material.

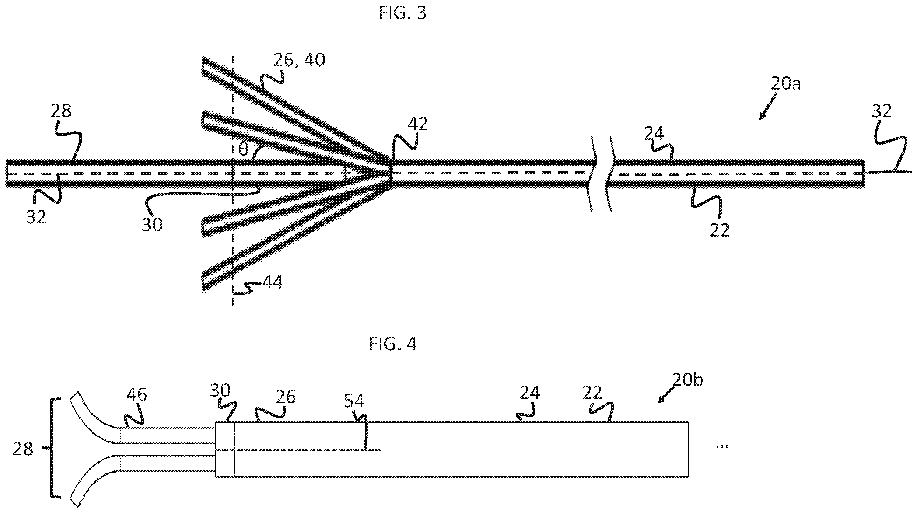

Wire 32 is configured to connect the more distal electrode to a power source 34 outside the subject's body; in other words, the proximal end of the wire is configured to connect to a terminal of power source 34. Similarly, another wire 38 is configured to connect the more proximal electrode to the other terminal of power source 34. For example, other wire 38 may pass through tube 24, with the proximal end of other wire 38 connected to the power source and the distal end of the other wire connected to the proximal electrode. Alternatively, as shown in Fig. 1, for embodiments in which tube 24 is electrically-conductive, the distal end of the other wire may be connected to tube 24, such that the other wire connects the proximal electrode to the power source via the tube. Alternatively, for embodiments in which the proximal electrode is elongated such that the proximal end of the proximal electrode remains outside the body, the other wire may connect to the proximal end of the proximal electrode. In any case, as further described below following the description of Fig. 3, power source 34 may apply a voltage between the two electrodes via wire 32 and other wire 38, thus facilitating the capture of the thrombus. (Equivalently, it may be said that the power source applies a voltage between the two wires, or between one of the wires and one of the electrodes.)

In apparatus 20, the (outer) diameter D1 of the reference electrode is larger (e.g., between 1.5 and 7 times larger, such as 3-5 times larger) than the (outer) diameter D2 of the active electrode, which is typically the same as, or only slightly greater than, the (outer) diameter of the tube. For example, diameter D1 may be between 0.35 and 3.5 mm, with diameter D2 being between 0.2 and 1.5 mm.

For example, each of the electrodes may comprise a tube or cylinder that covers tube 24, the wall of the reference electrode being thicker than that of the active electrode.

Alternatively, as shown in Figs. 1-2, each of the electrodes may comprise a tube or cylinder coupled distally to tube 24, the reference electrode having a diameter that is larger than that of the active electrode. In such embodiments, the diameter (equivalently, the circumference) of spacer 30 may vary along axis 36. In particular, for embodiments in which the reference electrode is distal to the active electrode (as in Fig. 1), the diameter of the spacer may be greater at the distal end of the spacer than at the proximal end of the spacer. For example, the proximal end of the spacer may have diameter D2, with the distal end of the spacer having diameter D1. Conversely, for embodiments in which the reference electrode is proximal to the active electrode (as in Fig. 2), the diameter of the spacer may be greater at the proximal end of the spacer than at the distal end of the spacer. For example, the proximal end of the spacer may have diameter D1, with the distal end of the spacer having diameter D2. Typically, in such embodiments, the diameter of the spacer varies linearly along axis 36, such that the spacer is cone-shaped.

Alternatively or additionally, the diameter of the reference electrode may vary (e.g., linearly) along axis 36. (For such embodiments, the diameter D1 described above may refer to the diameter of the reference electrode at any point at which the reference electrode is wider than the active electrode.) For example, for embodiments in which the reference electrode is distal to the active electrode, the diameter of the reference electrode may be greater at the distal end of the reference electrode than at the proximal end of the reference electrode. Alternatively or additionally, the diameter of the active electrode may vary (e.g., linearly) along axis 36.

Similarly, for embodiments in which the reference electrode is proximal to the active electrode, an intermediate portion 25 of the longitudinal element, belonging either to tube 24 or to reference electrode 26, may have a diameter that increases (e.g., linearly) moving distally along axis 36.

In other embodiments, the diameter of the reference electrode is the same, or approximately the same, as that of the active electrode. In such embodiments, instead of a larger-diameter reference electrode, the longitudinal element comprises a different larger-diameter portion, such as spacer 30 or another portion of tube 24. For example, both of the electrodes, along with the proximal and distal ends of the spacer, may have the smaller diameter D2, but a central portion of the spacer may have the larger diameter D1. Typically, in such embodiments, the larger-diameter portion of the longitudinal element is relatively close to the active electrode. For example, the larger-diameter portion of the longitudinal element may be disposed within 10 mm, such as within 5 mm, of the active electrode.

It is noted that, using an alternative descriptive scheme, the longitudinal element itself may be described as a multi-segmented tube, comprising (i) a first segment that functions as a reference electrode (or that is covered by a reference electrode), (ii) a second segment that functions as an active electrode (or that is covered by an active electrode), (iii) a third segment that electrically insulates the first and second segments from one another, and, optionally, (iv) a fourth segment that extends from the exterior of the body to the more proximal one of the first and second segments.

(It is noted that, for simplicity, power source 34, along with one or more of the wires that connect the power source to the electrodes, may be omitted from the subsequent figures.)

Reference is now made to Fig. 3, which is a schematic illustration of an apparatus 20a, comprising an expandable reference electrode, for removing a thrombus from a body of a subject, in accordance with some embodiments of the present disclosure.

Similarly to apparatus 20 (Figs. 1-2), apparatus 20a comprises tube 24, active electrode 28, and reference electrode 26. In apparatus 20a, however, the reference electrode does not have a fixed enlarged diameter. Rather, the diameter of the reference electrode is variable. In particular, when the reference electrode is constrained (e.g., by a constraining sheath), the reference electrode adopts a constrained configuration, in which the reference electrode is approximately flush with the tube. On the other hand, when not constrained, the reference electrode adopts an expanded configuration, in which the reference electrode radiates outwardly from the tube. Typically, the reference electrode is made from a shape memory material (e.g., nitinol) or a tempered material (e.g., tempered stainless steel or titanium), such that the reference electrode springs outwardly from the tube upon removal of the constraint.

In some embodiments, the reference electrode comprises a stent. In other embodiments, as shown in Fig. 3, the reference electrode comprises a plurality of (e.g., between three and 12, such as between four and nine) rods 40. The respective proximal ends of rods 40 are coupled to the tube, such that the rods expand from the tube by pivoting with respect to the tube. Rods 40 may be arranged, for example, in a circular arrangement along the circumference of the tube; for example, the respective proximal ends of the rods may be coupled to the tube at a circular rod-base 42. Typically, the rods are configured to expand outwardly from the tube such that, following a full expansion of the rods, the angle θ between each of the rods and the tube is between 10 and 60 degrees. (On the other hand, when the rods are constrained, angle θ is typically close to zero.) In some embodiments, the respective proximal portions of the rods are electrically insulated.

Typically, the reference electrode is configured to expand outwardly from the tube such that, following the expansion of the reference electrode, at least part of the reference electrode is opposite the active electrode. (In this context, the at least part of the reference electrode is considered to be "opposite" the active electrode in that there exists a hypothetical line radiating perpendicularly outward from the active electrode that intersects the reference electrode.) For example, in the expanded configuration of the rods, the respective distal portions of the rods - demarcated in Fig. 3 by a hypothetical line 44 that marks the boundary between spacer 30 and active electrode 28 - may be opposite the active electrode. Thus, when a voltage is applied between the electrodes, electric current (carried by ions) may flow radially between the electrodes, such that less current passes through the subject's tissue.

In some embodiments, as shown in Fig. 3, the active electrode is coupled to the tube distally to the reference electrode. For example, the active electrode may comprise a cylindrical element coupled distally to the tube. In some embodiments (such as embodiments in which tube 24 is electrically-conductive), spacer 30, which is typically of uniform diameter, is coupled distally to the tube between the active electrode and the tube, such that the active electrode is coupled distally to the tube via the spacer. Alternatively, the active electrode may comprise a coil, mesh, tube, or another element that covers tube 24.

In such embodiments, wire 32 (or another electrically-conductive element) is distally connected to the active electrode, and is configured to connect the active electrode to power source 34 (Fig. 1). Similarly, another wire is configured to connect the reference electrode to the power source, by connecting directly to the reference electrode or to tube 24.

In other embodiments, the reference electrode is coupled to the tube distally to the active electrode. For example, tube 24 may comprise two portions: a distal portion, to which the reference electrode is coupled, and a proximal portion disposed proximally to spacer 30. Active electrode 28 may comprise a cylindrical or tubular element disposed between the spacer and the proximal portion of the tube; alternatively, the active electrode may comprise a coil, mesh, tube, or another element that covers the proximal portion of the tube. Alternatively, the proximal portion of the tube itself may function as the active electrode.

In such embodiments, wire 32 is connected to the reference electrode, and another wire connects directly to the active electrode or to tube 24.

To remove a thrombus from a blood vessel using any of the embodiments in Figs. 1-3, the longitudinal element is inserted into the blood vessel, e.g., under fluoroscopy, and is advanced to the thrombus. For embodiments in which the reference electrode is expandable, the longitudinal element is inserted into the blood vessel while the reference electrode is constrained by a constraining force. For example, the reference electrode may be constrained by a constraining sheath that covers longitudinal element 22. For embodiments in which the reference electrode is distal to the active electrode, as in Fig. 1, the reference electrode may be passed through the thrombus.

Subsequently, the active electrode is positioned within the blood vessel such that a desired degree of contact between the active electrode and the thrombus is achieved. For example, the active electrode may be positioned within the thrombus, e.g., at or near the center of the thrombus.

Advantageously, the positioning of the active electrode may be facilitated by contacting the inner wall of the blood vessel with the reference electrode. For example, for embodiments in which the reference electrode is of a fixed larger diameter (as in Figs. 1-2), the active electrode may be positioned by advancing the active electrode toward the thrombus while the reference electrode acts as a bumper. In other words, by virtue of its greater diameter, the reference electrode may contact the inner wall of the blood vessel as the active electrode is advanced to the thrombus, thus inhibiting the active electrode from veering significantly from the center of the blood vessel.

Similarly, for embodiments in which the reference electrode is expandable, the constraining force may be removed from the reference electrode, such that the reference electrode expands outwardly from the tube. For example, the constraining sheath may be withdrawn from over the reference electrode, and/or the reference electrode may be advanced from the constraining sheath. Subsequently, the active electrode may be advanced to the thrombus while the reference electrode acts as a bumper.

Alternatively, a rough positioning may first be performed, whereby the active electrode is positioned within the thrombus or is aligned with the thrombus. Subsequently, the constraining force may be removed from the reference electrode, such that the reference electrode expands outwardly from the tube. As the reference electrode expands, the reference electrode may push against the inner wall of the blood vessel, thus further adjusting the position of the active electrode.

Subsequently to positioning the active electrode, a positive voltage is applied between the active electrode and the reference electrode, by applying a voltage between wire 32, which is connected to one of the electrodes, and other wire 38 (Fig. 1), which is connected to the other electrode. The application of the voltage causes the thrombus to adhere to the active electrode. Subsequently, while the thrombus adheres to the active electrode, the active electrode - along with the rest of the apparatus - is withdrawn from the subject's body.

### DEVICES FOR SIMULTANEOUSLY TREATING MULTIPLE BLOCKAGES

Reference is now made to Figs. 4-6, which are schematic illustrations of an apparatus 20b for removing multiple blockages, such as multiple thrombi, from respective branches 50 of a blood vessel 48 in a body of a subject, in accordance with some embodiments of the present disclosure.

As in the embodiments described above, apparatus 20b comprises longitudinal element 22, which typically comprises tube 24. However, in apparatus 20b, active electrode 28 comprises multiple electrode-wires 46, which are configured to extend distally from longitudinal element 22 into respective ones of branches 50.

In some embodiments, electrode-wires 46 are coupled distally to tube 24. In such embodiments, the electrode-wires may be connected to power source 34 (Fig. 1) by a single wire (or another electrically-conductive element) passing through the tube and distally connected to the electrode-wires. Alternatively, multiple connecting wires (or other electrically-conductive elements), each of which is distally connected to a different respective one of the electrode-wires, may pass through the tube.

In other embodiments, the electrode-wires themselves pass through tube 24. (Typically, in such embodiments, the electrode-wires are coupled to the inner wall of the tube.) In such embodiments, the electrode-wires may connect directly to the power source; alternatively, one or more connecting wires, which are disposed entirely outside the tube, may connect the electrode-wires to the power source. In such embodiments, the electrode-wires are typically insulated, with the exception of the distal portions of the electrode-wires that protrude from the tube.

Typically, tube 24 comprises an electrically-conductive portion, which functions as reference electrode 26, along with an electrically-insulative portion distal to the electrically-conductive portion, which functions as spacer 30. (In some embodiments, the electrically-conductive portion of the tube extends from the proximal end of the tube to the spacer.) Alternatively, the tube may be entirely electrically-insulative, and reference electrode 26 may comprise a tube, a mesh, a coil, or another element that covers the tube.

In some embodiments, as shown in Figs. 4-5, the active electrode comprises exactly two electrode-wires. Such embodiments may be used to treat an occluded bifurcation 52, in that each electrode-wire may be inserted into a different respective branch 50 of the bifurcation. Alternatively, the active electrode may comprise exactly three electrode-wires, for treatment of a trifurcation. Alternatively, the active electrode may comprise at least four (e.g., 4-100, such as 5-30) electrode-wires.

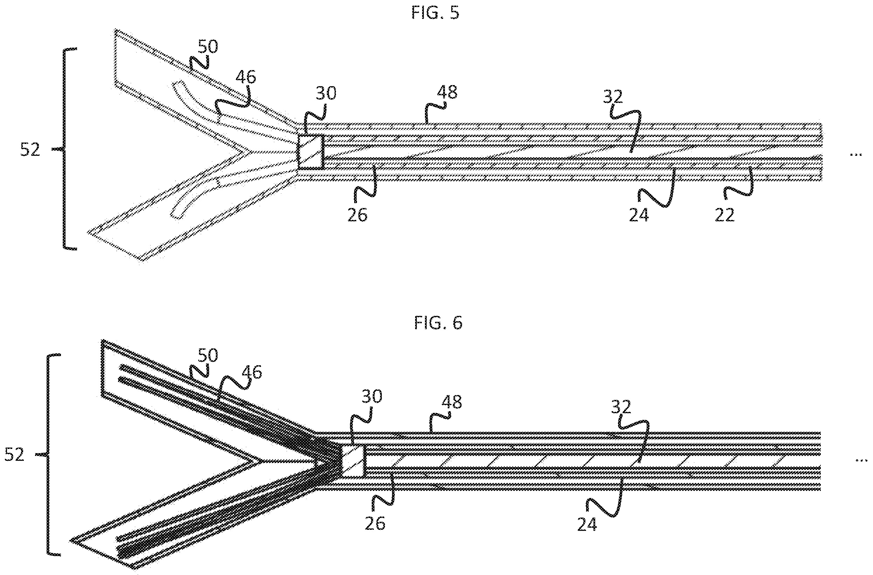

To remove the thrombi from branches 50, electrode-wires 46 are inserted into respective ones of branches 50. (As shown in Fig. 6, multiple electrode-wires may be inserted into the same branch.) Subsequently, a voltage is applied between the electrode-wires and the reference electrode, causing the thrombi to adhere to respective ones of the electrode-wires. Subsequently, while the thrombi adhere to the electrode-wires, the electrode-wires are withdrawn from the body.

In some embodiments, at least some of the electrode-wires are configured to bend toward the respective ones of the branches upon removal of a constraining force from the electrode-wires while the electrode-wires are positioned proximally to the branches. For example, at least some of the electrode-wires (or at least the distal portions thereof) may be made from a shape memory material, which may be pre-shaped into the desired bent form during the manufacturing process or at any subsequent time, e.g., immediately prior to the thrombectomy procedure. This bending facilitates the insertion of the electrode-wires into the branches, by increasing the probability that, as the electrode-wires are advanced toward the branches, at least one electrode-wire will enter each of the branches.

In such embodiments, prior to inserting the electrode-wires into the respective ones of the branches, the electrode-wires, while constrained by a constraining force into an unbent shape, are positioned proximally to the branches, typically such that the distal tips of the electrode-wires are within 10 mm from the branches. Subsequently, while the electrode-wires are positioned proximally to the branches, the constraining force is removed from the electrode-wires - e.g., by withdrawing a constraining sheath from over the electrode-wires - such that the electrode-wires bend toward the respective ones of the branches.

In some embodiments, as shown in Figs. 4-5, one or more of the electrode-wires are configured to bend toward the branches by curving toward the branches. For example, in the absence of a constraining force, the distal ends of the electrode-wires may each be curved, such that at least two of the distal ends are curved away from the central longitudinal axis 54 of tube 24 in different respective directions. In such embodiments, the distal end of at least one of the electrode-wires may be curved in a J-shape, such that the distal tip of the electrode-wire is approximately parallel to longitudinal axis 54.

Alternatively or additionally, one or more of the electrode-wires may be configured to bend toward the branches by angling toward the branches, rather than curving toward the branches. For example, in the absence of a constraining force, the electrode-wires may each bend (e.g., at an angle of at least 30 degrees) such that the respective distal ends of at least two of the electrode-wires angle away from central longitudinal axis 54 in different respective directions.

Alternatively or additionally, to increase the probability that at least one electrode-wire will enter each of the branches, apparatus 20b may comprise a relatively large number of electrode-wires, as in Fig. 6. Another advantage of such embodiments is that each thrombus may be contacted by multiple electrode-wires, thus increasing the electrostatic force with which the thrombus is held.

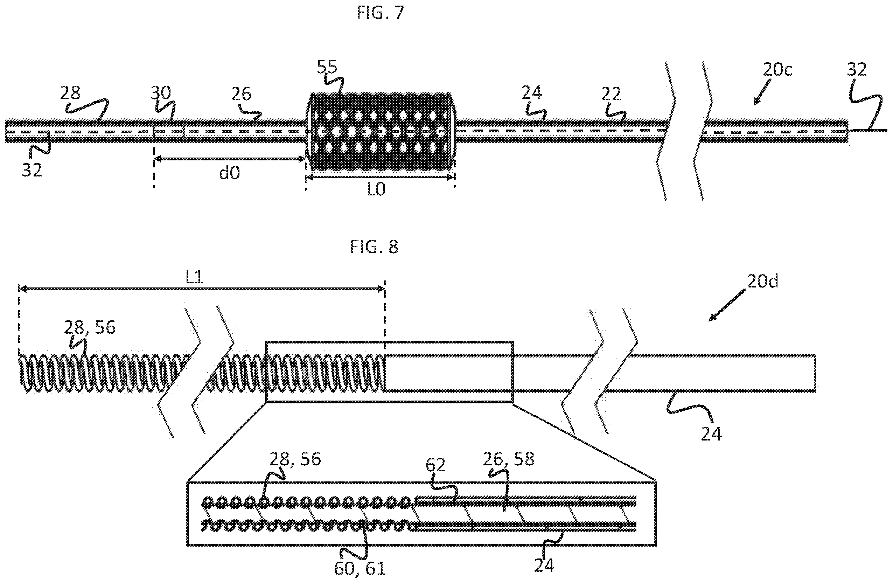

In other cases, multiple blockages, including a proximal blockage and a distal blockage, are disposed within the same branch of a blood vessel. In this regard, reference is now made to Fig. 7, which is a schematic illustration of an apparatus 20c for treating a proximal blockage (such as a dissection or calcification) and a distal blockage (such as a thrombus) in a blood vessel within a body of a subject, in accordance with some embodiments of the present invention.

Apparatus 20c comprises longitudinal element 22, which typically comprises tube 24. Active electrode 28 may be proximal or distal to reference electrode 26. In some embodiments, the electrodes are coupled distally to the tube, spacer 30 being disposed between the two electrodes. In other embodiments, the more proximal electrode, or both of the electrodes, comprise a coil, a mesh, or another element that covers the tube.

Apparatus 20c further comprises an expandable element 55, such as a stent, which may be made from any suitable biocompatible material such as stainless steel, nitinol, and/or titanium. Typically, the length L0 of the expandable element is between five and 100 mm, such as between 15 and 80 mm.

In some embodiments, expandable element 55 is detachably coupled to longitudinal element 22 proximally to active electrode 28. For example, expandable element 55 may be coupled to longitudinal element 22 over the reference electrode, such that the expandable element covers the reference electrode. Alternatively, for example, the expandable element may be coupled to tube 24 proximally to the reference electrode.

In such embodiments, to treat the blockages, longitudinal element 22 is inserted into the blood vessel, typically while contained within an outer sheath. The longitudinal element is then advanced through the blood vessel until expandable element 55 is disposed within the proximal blockage and the active electrode is within or near the distal blockage. During the insertion and advancement of the longitudinal element, the expandable element remains in a collapsed configuration, e.g., by virtue of being constrained by the outer sheath, and remains coupled to the longitudinal element.

Subsequently, expandable element 55 is expanded within the proximal blockage, e.g., by withdrawing the outer sheath and/or advancing the expandable element from the sheath. The expandable element thus adopts the expanded configuration shown in Fig. 7, in which the diameter of the expandable element is typically 2-15 times (e.g., 3-10 times) greater than the diameter of longitudinal element 22. Prior to, during, or subsequently to the expansion of the expandable element, while the expandable element is within the proximal blockage, the expandable element is detached from the longitudinal element using any suitable detachment mechanism, such as an electrolytic or a mechanical detachment mechanism. The expandable element thus remains in place within the blood vessel, inhibiting occlusion of the blood vessel by the proximal blockage.

Additionally, prior to, during, or subsequently to the expansion of the expandable element, a voltage is applied between the active electrode and the reference electrode, causing the distal blockage to adhere to the active electrode. Subsequently, the active electrode, together with the distal blockage, are withdrawn from the body. (As the active electrode is withdrawn, the active electrode passes through the expandable element.)

Typically, the distance d0 between the distal end of the expandable element and the proximal end of the active electrode is between five and 100 mm, such as between 30 and 70 mm; this range corresponds to the typical distance between two blockages in a blood vessel.

In other embodiments, the expandable element is detachably coupled to the distal end of a separate insertion tube, rather than being coupled to the longitudinal element. In such embodiments, the expandable element may be inserted into the blood vessel and then advanced through the blood vessel until the expandable element is positioned with the proximal blockage. Subsequently, the longitudinal element may be inserted, through the insertion tube, into the blood vessel. (Thus, the longitudinal element is contained within the insertion tube, which is in turn contained within the outer sheath.) The longitudinal element may then be passed through the expandable element until the active electrode is positioned within or near the distal blockage. Advantageously, the expandable element may be configured such that even while constrained, the expandable element provides a lumen having a diameter of at least 0.3 mm, so as to facilitate passage of the longitudinal element therethrough.

Alternatively, the longitudinal element may be inserted into the body, while contained within the insertion tube, together with the expandable element. Subsequently to positioning the expandable element at the proximal blockage, the longitudinal element may be passed through the expandable element as described above.

Subsequently, the blockages may be treated by expanding and detaching the expandable element, and also applying a voltage between the electrodes. As described above, the expandable element may be detached using any suitable detachment mechanism.

As an alternative, the expandable element may be expanded and detached from the insertion tube before the longitudinal element is passed through the expandable element.

In such embodiments, distance d0 is adjustable by virtue of the fact that, as described above, the longitudinal element may be passed proximally or distally through the expandable element. Thus, distance d0 may be adjusted prior to expanding the expandable element, e.g., by advancing or withdrawing the longitudinal element through the expandable element, and/or by advancing or withdrawing the insertion tube. For example, an image of the blockages may be acquired, and the distance between the blockages may be ascertained from the image. Subsequently, distance d0 may be set to the distance between the blockages, such that, when the expandable element is within the proximal blockage, the active electrode is within, or parallel to, the distal blockage.

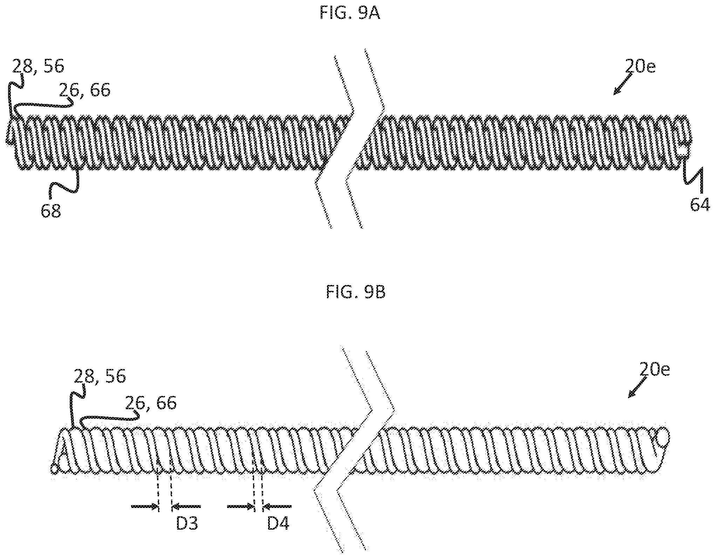

It is noted that expandable element 55 may be combined with any of the other embodiments described herein. For example, the scope of the present invention includes a thrombectomy device comprising expandable element 55 along with an enlarged or expandable reference electrode (Figs. 1-3), multiple active electrode-wires (Figs. 4-6), or at least one coiled electrode (Figs. 8 and 9A-B).

### DEVICES HAVING COILED ELECTRODES

Reference is now made to Fig. 8, which is a schematic illustration of an apparatus 20d comprising a coiled active electrode for removing a blockage from a body of a subject, in accordance with some embodiments of the present disclosure.

In apparatus 20d, reference electrode 26 comprises a wire 58, a distal portion of which is partly covered by an electrically-insulative element 60. Active electrode 28 comprises a coil 56, which is coiled over electrically-insulative element 60, without contacting the wire, such that at least one exposed portion of the wire (i.e., at least one portion of the wire not covered by the electrically-insulative element) is disposed between successive windings of the coil or underneath the coil. Thus, while a voltage is applied between the coil and the wire, electric current (carried by ions) flows between the coil and the exposed portions of the wire.

In some embodiments, electrically-insulative element 60 is attached to the wire, e.g., by an adhesive or any suitable coating or heat-shrinking process. Alternatively or additionally, the electrically-insulative element may be attached to the coil, e.g., via any of the aforementioned techniques.

In some embodiments, electrically-insulative element 60 comprises an electrically-insulative strip 61 wound over the distal portion of wire 58 such that the distal portion of the wire is exposed between at least some windings of strip 61. In some such embodiments, strip 61 has a relatively large pitch such that, for example, at least 50% of the distal portion of the wire is exposed between the windings of the strip. In other embodiments, the pitch may be smaller such that, for example, less than 50%, e.g., less than 30%, is exposed. In other embodiments, the electrically-insulative element comprises a plurality of rings spaced apart from each other along the distal portion of the wire, such that the distal portion of the wire is exposed between the rings.

Typically, the length L1 of the coil, which is typically the same as the length of the distal portion of wire 58, is less than 150 mm, such as between five and 50 mm.

Apparatus 20d further comprises an electrically-conductive element that is distally connected to the coil, and is configured to connect the coil to power source 34 (Fig. 1). In some embodiments, the electrically-conductive element comprises tube 24. In particular, as shown in Fig. 8, the tube may be distally connected to the coil, such that the tube may connect the power source to the coil via a connecting wire extending from the proximal end of tube to the power source. In other embodiments, the electrically-conductive element comprises a connecting wire distally connected to the coil. This connecting wire may pass through tube 24; alternatively, apparatus 20d may not comprise tube 24, and the connecting wire may be coupled to insulating cover 62.

Typically, the portion of wire 58 proximal to coil 56 is covered by an insulating cover 62, such that wire 58 is insulated from tube 24 and/or from any other electrically-conductive elements. In some embodiments, electrically-insulative element 60 is continuous with insulating cover 62, i.e., a single piece of insulating material comprises both electrically-insulative element 60 and insulating cover 62.

Typically, for embodiments comprising tube 24, wire 58 is coupled to the inner wall of tube 24. Thus, typically, the wire is not slidable within the tube, but rather, is held in place such that the distal portion of the wire, which is covered by coil 56, protrudes from the tube.

To remove a thrombus using apparatus 20d, wire 58 is inserted into the blood vessel, and is advanced through the blood vessel until coil 56 is disposed within or adjacent to the thrombus. Subsequently, a voltage is applied between coil 56 and wire 58, such that the thrombus adheres to the coil. The thrombus is then removed from the blood vessel.

Reference is now made to Figs. 9A-B, which are schematic illustrations of an apparatus 20e comprising a coiled active electrode and coiled reference electrode for removing a blockage from a body of a subject, in accordance with some embodiments of the present invention.

In apparatus 20e, as in apparatus 20d (Fig. 8), active electrode 28 comprises coil 56, which is referred to hereinbelow as an "active coil." Apparatus 20e differs from apparatus 20d, however, in that reference electrode 26 also comprises a coil, referred to herein as a "reference coil" 66. Active coil 56 and reference coil 66 are both wound (i.e., the electrodes are co-wound) around an electrically-insulative longitudinal core 64, which is visible in Fig. 9A. An electrically-insulative element (or "layer") 68, such as a strip or another coil made of an insulating material, interposes between the active coil and the reference coil. Each of the coils may be connected, via a respective wire, to power source 34 (Fig. 1). Typically, at least one of the coils is insulated over most of its length, with only the distal portion of the coil being uninsulated.

In some embodiments, core 64 comprises a plastic wire or an insulated metal wire. In other embodiments, core 64 is hollow, such that a tool, a contrast material, or any other relevant item may be passed through the core. For example, core 64 may comprise a plastic tube or an insulated metal tube.

In some embodiments, the active coil and/or the reference coil does not cover the full length of core 64, but rather, only a distal portion thereof. For example, the length of the active coil and/or the reference coil may be between 5 and 100 mm, such that the coil covers only the distalmost 5-100 mm of the longitudinal core.

In some embodiments, as shown in Figs. 9A-B, the active coil and the reference coil are wound next to one another, such that the windings of one coil alternate with those of the other coil. (Typically, in such embodiments, each of the coils contacts the electrically-insulative core.) In such embodiments, as shown in Fig. 9B, the diameter D3 of the active coil may be greater (e.g., 1.5-6 times greater, such as 2-4 times greater) than the diameter D4 of the reference coil, in accordance with the invention as claimed in claim 1. For example, diameter D3 may be between 0.075 and 0.35 mm, with diameter D4 being between 0.05 and 0.2 mm. Advantageously, the greater diameter of the active coil may provide an increased area for contact with the thrombus, and hence, a greater attractive force applied to the thrombus. Alternatively or additionally, to increase the contact area, the pitch of the active coil and the reference coil may be minimized such that little or none of the core is exposed, at least near the distal end of the longitudinal core. For example, at or near the distal end of the longitudinal core, the active coil and the reference coil may completely cover the longitudinal core for a length of at least 5 mm, such as a length of at least 10 mm. As an example of this, the active coil and the reference coil may cover the entirety of the core, as shown in Fig. 9B.

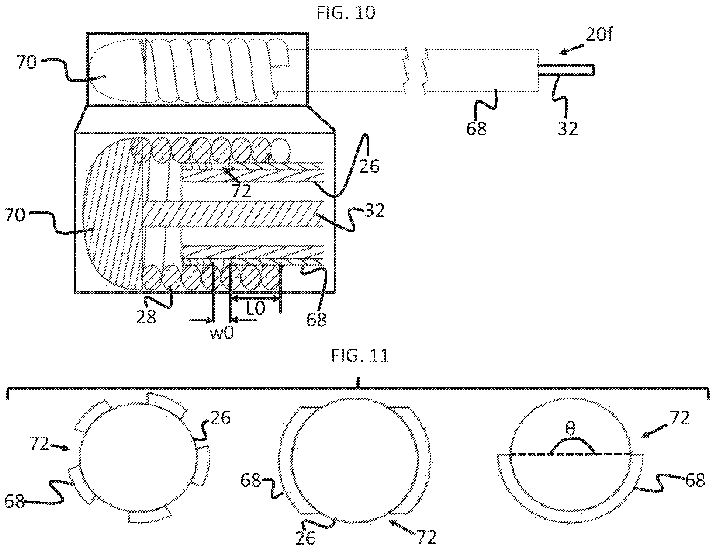

In other embodiments, the active coil is wound over the reference coil, in accordance with the invention as claimed in claim 12, such that the reference coil and electrically-insulative element 68 interpose between the active coil and the longitudinal core. For example, as further described below with reference to Fig. 10, the longitudinal core may comprise an insulated wire configured to electrically connect the active coil to the power source, the reference coil may be wound around the insulated wire (not necessarily in contact with the insulated wire), the electrically-insulative element may cover the reference coil, and the active coil may be wound over the electrically-insulative element.

To remove a thrombus from a blood vessel using apparatus 20e, the apparatus is inserted into the blood vessel, and is then advanced through the blood vessel until the active coil is disposed within or adjacent to the thrombus. Subsequently, a positive voltage is applied between the active coil and the reference coil, causing the thrombus to adhere to the active coil. Subsequently, while the thrombus adheres to the active coil, the apparatus is withdrawn from the body of the subject.

For embodiments in which core 64 is hollow, a tool or contrast agent may be passed through the core prior to, subsequently to, or during the application of the voltage. For example, a net may be passed through the core prior to the withdrawal of the apparatus from the body, such that the net protrudes from the distal end of the core. Subsequently, as the apparatus and the net are withdrawn, the net may catch any detached fragments from the thrombus.

Reference is now made to Fig. 10, which is a schematic illustration of another apparatus 20f for removing a blockage from a body of a subject, in accordance with some embodiments of the present disclosure. Reference is further made to Fig. 11, which is a schematic illustration of various transverse cross-sectional cuts through apparatus 20f, in accordance with some embodiments of the present disclosure.

In apparatus 20f, as in some embodiments of apparatus 20e described above with reference to Figs. 9A-B, the active electrode is disposed over the reference electrode, with electrically-insulative element 68 interposing between the two electrodes (i.e., the electrically-insulative element covers the reference electrode, and the active electrode covers the electrically-insulative element). Electrically-insulative element 68 is shaped to define at least one gap 72 that exposes a portion of the reference electrode. During the application of voltage between the two electrodes, electric current (carried by ions) flows between the two electrodes via gap 72, thus facilitating the capture of the thrombus. Typically, the width w0 of the gap (i.e., the distance between the proximal and distal ends of the gap) is between 0.01 and 1 mm, such as between 0.05 and 0.5 mm.

Wire 32 (or another electrically-conductive element), which is typically insulated over most of its length, passes through the reference electrode and is electrically connected to the active electrode. For example, the wire may be distally connected to an electrically-conductive cap 70 connected to the distal end of the active electrode. Cap 70 may be disposed at a small distance from the distal end of the electrically-insulative element, such that the distal end of the active electrode, which is connected to the cap, protrudes beyond the electrically-insulative element.

Typically, as shown in Fig. 10, the active electrode comprises a coil. Alternatively, the active electrode may comprise a mesh or tube, or may have any other suitable form. Typically, the active electrode does not extend over the full length of apparatus 20f, but rather, has a length of between 5 and 100 mm.

In some embodiments, as shown in Fig. 10, the reference electrode comprises a tube. Alternatively, the reference electrode may comprise a tube, a mesh, or any other suitable structure.

In some embodiments, the electrically-insulative element comprises a strip of electrically-insulative material disposed underneath the active electrode. In other embodiments, the electrically-insulative element comprises a tube. In such embodiments, as shown in Fig. 10, the electrically-insulative element may extend from the proximal end of apparatus 20f to the distal end of the apparatus, such that the electrically-insulative element provides rigidity to the apparatus. Alternatively, the electrically-insulative element may be disposed only at the distal end of the apparatus, such that, for example, the proximal end of the electrically-insulative element is disposed underneath, or only slightly proximal to, the proximal end of the active electrode. In such embodiments, rigidity may be provided by reference electrode 26.

Typically, the active electrode is attached (e.g., via an adhesive) to the electrically-insulative element, at least at one or both ends of the active electrode.

In some embodiments, the reference electrode is attached to the electrically-insulative element. Alternatively or additionally, the reference electrode may be attached to wire 32; for example, the reference electrode may comprise a coil or mesh that is wrapped around the wire. In any case, typically, the respective positions of the electrodes are fixed relative to one another.

In some embodiments, gap 72 extends around the full circumference of the reference electrode. In other embodiments, as shown in the rightmost example in Fig. 11, the gap - which, in these embodiments, may be referred to as a "perforation" - extends around only part of the circumference. In such embodiments, the angular span θ of the gap may be between 150 and 210 degrees, such as between 170 and 190 degrees. Optionally, as shown in the leftmost and middle examples in Fig. 11, multiple such gaps (each having any suitable angular span) may be disposed at different respective positions along the circumference of the reference electrode.

In some embodiments, multiple gaps are disposed at different respective positions along the longitudinal axis of the reference electrode, i.e., some of the gaps may be proximal or distal to each other. In such embodiments, the distance between successive gaps is typically greater than 0.1 mm, e.g., greater than 1 mm, for improved distribution of the electric current.

Typically, the active electrode covers the electrically-insulative element both proximally and distally to each gap 72. Further typically, the distance L0 between the proximal end of the active electrode and the most proximal gap is around half the length of the thrombus that is to be removed, such as between 0.25 and 0.75 times (e.g., between 0.4 and 0.6 times) the length of the thrombus. (In this context, the "length" of the thrombus refers to the distance between the proximal and distal ends of the thrombus.)

As demonstrated in the experiment described below, the attractive force between the active electrode and the thrombus is a decreasing function of the distance of the thrombus from the nearest exposed portion of the reference electrode. During the thrombectomy procedure, the physician typically aligns the proximal end of the active electrode with the proximal end of the thrombus. Accordingly, setting L0 to around half of the length of the thrombus generally minimizes the average distance of the thrombus from the gap, and hence, generally maximizes the attractive force between the active electrode and the thrombus.

The present inventors have observed that the length of a thrombus is typically 1-3 times the diameter of the blood vessel in which the thrombus is located. Accordingly, L0 may be around 0.5-1.5 times the diameter of the blood vessel into which the apparatus is to be inserted. By way of example, Table 1 below lists example ranges for L0 for various types of blood vessels. In particular, for each type of blood vessel, the table specifies a range of expected diameters. The lower end of the range for L0 is 0.5 times the lowest expected diameter, while the upper end of the range is 1.5 times the greatest expected diameter.

**Table 1**

| Blood vessel | Expected diameter [mm] | L0 [mm] |
|---|---|---|
| Middle cerebral artery | 1-4 | 0.5-6 |
| Internal carotid artery | 4-6 | 2-9 |
| Basilar artery | 1-5 | 0.5-7.5 |
| Pulmonary artery | 10-18 | 5-27 |
| Iliac vein | 4-10 | 2-15 |
| External iliac and common femoral veins | 5-14 | 2.5-21 |
| Superficial femoral, deep femoral and popliteal veins | 4-10 | 2-15 |
| Calf veins | 4-8 | 2-12 |

Some embodiments of the present disclosure provide a kit of apparatuses 20f, each apparatus having a different respective distance L0. For example, one apparatus may have an L0 of 1 mm, another an L0 of 2 mm, etc. Prior to the procedure, the physician may estimate (e.g., based on a fluoroscopic image) the diameter of the blood vessel and/or the length of the thrombus, and then select the appropriate apparatus responsively thereto. In the event that there is a significant degree of uncertainty in the physician's estimate, the physician typically matches the apparatus to the smallest estimated diameter or length. For example, for a thrombus having an estimated length of 4-6 mm, the physician may select an apparatus having an L0 of 2 mm, which is half of the lower end of the range.

Alternatively, each apparatus in the kit may be designated for a different respective type of blood vessel, such that the physician need not perform any estimation prior to selecting the appropriate apparatus. For example, an apparatus designated for the middle cerebral artery may have an L0 of 3.25 mm, which is the mean of the relevant range in the table above.

To remove a thrombus from a blood vessel using apparatus 20f, the apparatus is inserted into the blood vessel, and is then advanced through the blood vessel until the active electrode is disposed within or adjacent to the thrombus. Subsequently, a positive voltage is applied between wire 32 and the reference electrode, causing the thrombus to adhere to the active electrode. Subsequently, while the thrombus adheres to the active electrode, the apparatus is withdrawn from the body of the subject.

### EXPERIMENTAL RESULTS

The inventors conducted an experiment to explore the dependency of the attractive force between the active electrode and the thrombus on the distance of the thrombus from the nearest exposed portion of the reference electrode. For this experiment, the inventors used a device comprising (i) an electrically-insulative tube, (ii) a gold wire passing through the tube and protruding from the distal end of the tube, and (ii) a titanium coil coiled around the electrically-insulative tube such that the distal end of the titanium coil was positioned 1 mm from the distal end of the electrically-insulative tube.

A clot of swine blood was placed in a silicone tube filled with saline. Subsequently, the gold wire was passed through the clot, a voltage between the gold wire and the titanium coil was applied, and the resulting attractive force between the gold wire and the clot was measured. (The force was measured by connecting the device to a force gauge by a mechanical vise, and slowly removing the clot with tweezers.) This procedure was repeated seven times for each of a plurality of different distances between the proximal end of the clot and the distal end of the electrically-insulative tube.

Given that the thickness of electrically-insulative element 68 is generally around 1 mm, the distance between the proximal end of the clot and the distal end of the electrically-insulative tube is analogous to distance L0 (provided that, as described above, the proximal end of the active electrode is aligned with the proximal end of the thrombus). Hence, this experiment simulated using apparatus 20f with various different L0 values.

The results of the experiment, shown in Table 2 below, demonstrate that the attractive force is a decreasing function of the distance of the thrombus from the nearest exposed portion of the reference electrode, and hence, that it is advantageous to vary L0 in accordance with the length of the thrombus.

**Table 2**

| Distance between clot and tube (mm) | Mean force (mN) | Standard deviation of force (nM) |
|---|---|---|
| 1 | 22.8 | 10.6 |
| 2 | 19.2 | 11.4 |
| 3 | 15.9 | 14.6 |
| 4 | 9.1 | 9.0 |
| 5 | 5.7 | 5.7 |

It will be appreciated by persons skilled in the art that the present invention is not limited to what has been particularly shown and described hereinabove. Rather, the scope of the present invention is defined by the following claims.

## Claims

1. Apparatus (20e, 20f) for removing a blockage from a body of a subject, the apparatus comprising:
a longitudinal core (64), configured for insertion into the body;
an active coil electrode (56) and a reference coil electrode (66), which are wound next to one another over the longitudinal core, wherein an active-coil diameter of the active coil electrode is greater than a reference-coil diameter of the reference coil electrode; and
an electrically-insulative element (68) that interposes between the active coil electrode and the reference coil electrode;
the active coil electrode being configured to attract the blockage upon application of a voltage, by a power source (34), between the active coil electrode and the reference coil electrode.

2. The apparatus according to claim 1, wherein the longitudinal core comprises an insulated wire configured to electrically connect the active coil electrode to the power source.

3. The apparatus according to claim 1, wherein the longitudinal core is hollow.

4. The apparatus according to claim 3, further comprising a tool configured to pass through the longitudinal core.

5. The apparatus according to claim 4, wherein the tool comprises a net configured to catch any detached fragments of the blockage.

6. The apparatus according to claim 3, further comprising a contrast agent configured to pass through the longitudinal core.

7. The apparatus according to any one of claims 1-6, wherein the electrically-insulative element comprises a strip.

8. The apparatus according to any one of claims 1-6, wherein the electrically-insulative element comprises another coil.

9. The apparatus according to any one of claims 1 to 8, wherein the active-coil diameter is 1.5-6 times greater than the reference-coil diameter.

10. The apparatus according to claim 9, wherein the active-coil diameter is 2-4 times greater than the reference-coil diameter.

11. The apparatus according to any one of claims 1-10, wherein the active coil and the reference coil completely cover the longitudinal core for a length of at least 5 mm.

12. Apparatus (20e, 20f) for removing a blockage from a body of a subject, the apparatus comprising:
a longitudinal core (64), configured for insertion into the body;
a reference coil electrode (66) wound over the longitudinal core;
an active coil electrode (56) wound over the reference coil electrode (66); and
an electrically-insulative element (68) that interposes between the active coil electrode and the reference coil electrode;
the active coil electrode being configured to attract the blockage upon application of a voltage, by a power source (34), between the active coil electrode and the reference coil electrode.

13. The apparatus according to claim 12, wherein the longitudinal core comprises an insulated wire configured to electrically connect the active coil electrode to the power source.

14. The apparatus according to claim 12, wherein the longitudinal core is hollow.

15. The apparatus according to claim 14, further comprising a tool configured to pass through the longitudinal core.

## Patentansprüche

1. Vorrichtung (20e, 20f) zum Entfernen einer Blockade aus einem Körper eines Subjekts, die Vorrichtung umfassend:
einen Längskern (64), der zum Einsetzen in den Körper konfiguriert ist;
eine aktive Spulenelektrode (56) und eine Referenzspulenelektrode (66), die nebeneinander über den Längskern gewickelt sind, wobei ein aktiver Spulendurchmesser der aktiven Spulenelektrode größer ist als ein Referenzspulendurchmesser der Referenzspulenelektrode; und
ein elektrisch isolierendes Element (68), das zwischen die aktive Spulenelektrode und die Referenzspulenelektrode eingefügt ist;
wobei die aktive Spulenelektrode konfiguriert ist, um die Blockade bei Anlegen einer Spannung durch eine Stromquelle (34) zwischen der aktiven Spulenelektrode und der Referenzspulenelektrode anzuziehen.

2. Vorrichtung nach Anspruch 1, wobei der Längskern einen isolierten Draht umfasst, der konfiguriert ist, um die aktive Spulenelektrode elektrisch mit der Stromquelle zu verbinden.

3. Vorrichtung nach Anspruch 1, wobei der Längskern hohl ist.

4. Vorrichtung nach Anspruch 3, ferner umfassend ein Instrument, das konfiguriert ist, um durch den Längskern zu verlaufen.

5. Vorrichtung nach Anspruch 4, wobei das Instrument ein Netz umfasst, das konfiguriert ist, um beliebige abgelöste Fragmente der Blockage einzufangen.

6. Vorrichtung nach Anspruch 3, ferner umfassend ein Kontrastmittel, das konfiguriert ist, durch den Längskern zu verlaufen.

7. Vorrichtung nach einem der Ansprüche 1-6, wobei das elektrisch isolierende Element einen Streifen umfasst.

8. Vorrichtung nach einem der Ansprüche 1-6, wobei das elektrisch isolierende Element eine weitere Spule umfasst.

9. Vorrichtung nach einem der Ansprüche 1 bis 8, wobei der Durchmesser der aktiven Spule 1,5-6 mal größer ist als der Durchmesser der Referenzspule.

10. Vorrichtung nach Anspruch 9, wobei der Durchmesser der aktiven Spule 2-4 mal größer ist als der Durchmesser der Referenzspule.

11. Vorrichtung nach einem der Ansprüche 1-10, wobei die aktive Spule und die Referenzspule den Längskern über eine Länge von mindestens 5 mm vollständig bedecken.

12. Vorrichtung (20e, 20f) zum Entfernen einer Blockade aus einem Körper eines Subjekts, die Vorrichtung umfassend:
einen Längskern (64), der zum Einsetzen in den Körper konfiguriert ist;
eine Referenzspulenelektrode (66), die über den Längskern gewickelt ist;
eine aktive Spulenelektrode (56), die über die Referenzspulenelektrode (66) gewickelt ist; und
ein elektrisch isolierendes Element (68), das zwischen die aktive Spulenelektrode und die Referenzspulenelektrode eingefügt ist;
wobei die aktive Spulenelektrode konfiguriert ist, um die Blockade bei Anlegen einer Spannung durch eine Stromquelle (34) zwischen der aktiven Spulenelektrode und der Referenzspulenelektrode anzuziehen.

13. Vorrichtung nach Anspruch 12, wobei der Längskern einen isolierten Draht umfasst, der konfiguriert ist, um die aktive Spulenelektrode elektrisch mit der Stromquelle zu verbinden.

14. Vorrichtung nach Anspruch 12, wobei der Längskern hohl ist.

15. Vorrichtung nach Anspruch 14, ferner umfassend ein Instrument, das konfiguriert ist, um durch den Längskern zu verlaufen.

## Revendications

1. Appareil (20e, 20f) pour éliminer un blocage du corps d'un sujet, l'appareil comprenant :
un noyau longitudinal (64), configuré pour être inséré dans le corps ;
une électrode de bobine active (56) et une électrode de bobine de référence (66), qui sont enroulées l'une à côté de l'autre sur le noyau longitudinal, dans lequel un diamètre de bobine active de l'électrode de bobine active est supérieur à un diamètre de bobine de référence de l'électrode de bobine de référence ; et
un élément électriquement isolant (68) qui s'interpose entre l'électrode de bobine active et l'électrode de bobine de référence ;
l'électrode de bobine active étant configurée pour attirer le blocage lors de l'application d'une tension, par une source d'alimentation (34), entre l'électrode de bobine active et l'électrode de bobine de référence.

2. Appareil selon la revendication 1, dans lequel le noyau longitudinal comprend un fil isolé configuré pour connecter électriquement l'électrode de bobine active à la source d'alimentation.

3. Appareil selon la revendication 1, dans lequel le noyau longitudinal est creux.

4. Appareil selon la revendication 3, comprenant en outre un outil configuré pour passer à travers le noyau longitudinal.

5. Appareil selon la revendication 4, dans lequel l'outil comprend un filet configuré pour attraper tout fragment détaché du blocage.

6. Appareil selon la revendication 3, comprenant en outre un agent de contraste configuré pour passer à travers le noyau longitudinal.

7. Appareil selon l'une quelconque des revendications 1 à 6, dans lequel l'élément électriquement isolant comprend une bande.

8. Appareil selon l'une quelconque des revendications 1 à 6, dans lequel l'élément électriquement isolant comprend une bobine.

9. Appareil selon l'une quelconque des revendications 1 à 8, dans lequel le diamètre de la bobine active est 1,5 à 6 fois supérieur au diamètre de la bobine de référence.

10. Appareil selon la revendication 9, dans lequel le diamètre de la bobine active est 2 à 4 fois supérieur au diamètre de la bobine de référence.

11. Appareil selon l'une quelconque des revendications 1 à 10, dans lequel la bobine active et la bobine de référence recouvrent complètement le noyau longitudinal sur une longueur d'au moins 5 mm.

12. Appareil (20e, 20f) pour éliminer un blocage du corps d'un sujet, l'appareil comprenant :
un noyau longitudinal (64), configuré pour être inséré dans le corps ;
une électrode de bobine de référence (66) enroulée sur le noyau longitudinal ;
une électrode de bobine active (56) enroulée sur l'électrode de bobine de référence (66) ; et
un élément électriquement isolant (68) qui s'interpose entre l'électrode de bobine active et l'électrode de bobine de référence ;
l'électrode de bobine active étant configurée pour attirer le blocage lors de l'application d'une tension, par une source d'alimentation (34), entre l'électrode de bobine active et l'électrode de bobine de référence.

13. Appareil selon la revendication 12, dans lequel le noyau longitudinal comprend un fil isolé configuré pour connecter électriquement l'électrode de bobine active à la source d'alimentation.

14. Appareil selon la revendication 12, dans lequel le noyau longitudinal est creux.

15. Appareil selon la revendication 14, comprenant en outre un outil configuré pour passer à travers le noyau longitudinal.
